# EUROPEAN PATENT APPLICATION

(11) **EP 4 789 693 A1**
(43) Date of publication of application: **12.08.2026**
(21) Application number: 25156769.9
(22) Date of filing: 10.02.2025
(51) Int. Cl.: A61L 27/36

(54) **MAMMALIAN SPLIT-THICKNESS SKIN GRAFT MATERIAL FOR PROMOTING ATTACHED GINGIVA AND/OR MUCOSA GROWTH AND/OR HEALING**

(71) Applicant: Cacaci, Claudio, 82049 Pullach i. Isartal (DE)
(72) Inventor: Cacaci, Claudio, 82049 Pullach i. Isartal (DE)
(74) Representative: Krauss, Jan

(57) **Abstract**

The present invention relates to a non-autologous mammalian split-thickness skin graft material for use in the treatment of a subject with deficient and/or missing attached gingiva. Further provided is a kit comprising the non-autologous mammalian split-thickness skin graft material and uses thereof.

## Description

### FIELD OF THE INVENTION

The present invention relates to a non-autologous mammalian split-thickness skin graft material for use in the treatment of a subject with deficient and/or missing attached gingiva (keratinized gingiva). Further provided is a kit comprising the non-autologous mammalian split-thickness skin graft material and uses thereof.

### DESCRIPTION

Gingiva is basically divided into two parts, attached and moveable gingiva. The attached gingiva (keratinized gingiva) extends from the tooth/implant sulcus to the mucogingival junction, and the movable gingiva extends from the mucogingival junction into the vestibulum (1). Attached gingiva (keratinized gingiva) plays a crucial role in maintaining the health of teeth and implants since its absence can lead to inflammatory processes, ultimately resulting in the loss of the tooth or the implant (2, 3). Current clinical studies report that a thickness/width of least ≥ 2 mm of attached gingiva (keratinized gingiva) around implants and teeth is necessary to enable healthy teeth and implants (4).

To date, the favored approach to addressing an attached gingiva (keratinized gingiva) deficiency is an apically positioned flap/vestibuloplasty procedure, either with or without a graft combination (5). Consensus exists regarding the significant reduction of shrinkage with the use of grafts (6, 7). Autologous tissue grafts from the oral cavity, known as free gingival grafts from the palate, are considered the gold standard to generate or increase attached gingiva (keratinized gingiva), but they are associated with significant donor site morbidity, have a limitation in size and availability and they require additional surgical time for harvesting.

Processed allogeneic, e.g. from another human donor, or xenogeneic substitutes come with an unpredictable efficacy in the increase of attached gingiva (keratinized gingiva), and exhibit inferior outcome when compared with autologous grafts, while synthetic materials exhibit even lower efficacy than xenogeneic grafts in clinical studies (8). Therefore, a lack of attached gingiva (keratinized gingiva) cannot be treated sufficiently with existing soft tissue substitutes.

In the early 1960s, Slanetz and Rankow reported positive outcomes from extraoral autologous split-thickness skin transfer into the oral cavity to cover large mucosal defects in humans resulting from trauma or tumor surgery (9). The skin such as dermatome is typically harvested from the anterior thigh at a thickness of 0.2-0.5 mm using appropriate cutting instruments.

Therefore, a split-thickness skin graft, by definition, refers to a graft that contains the epidermis and a portion of the dermis, which is in contrast to a full-thickness skin graft consisting both of the epidermis and entire dermis.

While the use of autogenous grafts like split-skin or meshed-skin grafts from extraoral areas is widely accepted, the donor site morbidity remains a concern. Fang et al. describe the transplantation of autologous split-thickness skin from the thigh after microvascular anastomosed bone reconstructions as a feasible and safe solution to create attached gingiva (keratinized gingiva) in preparation of implant-prosthetic reconstruction (10). Consequently, autologous split-thickness transplantation is performed when the availability of free gingival grafts is limited. This approach is typically reserved for addressing substantial soft tissue defects in patients during reconstruction after tumor resection, trauma or advanced bone grafting procedures (6, 7).

Porcine full-thickness and split-thickness skin grafts were used for large-area skin burns when autologous material is insufficient to cover the areas (11, 12). Porcine lyophilized split-thickness skin grafts (0.3 - 0.5 mm) have also been described to cover oral mucosal defects in dogs and humans (13, 14). However, the use of porcine split-thickness skin grafts for the generation of attached gingiva (keratinized gingiva) in the oral cavity has not been described yet.

It is an object of the present invention to provide an improved approach for overcoming the described attached gingiva (keratinized gingiva) deficiencies. Other objects and advantages of the present invention will become apparent to the person skilled in the art when studying the following more detailed description of the present invention, including the Figures and examples.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

According to a first aspect of the present invention, the above object is solved by providing a non-autologous mammalian split-thickness skin graft material for use in the treatment of a deficient and/or missing attached gingiva (keratinized gingiva) and/or mucosa in a subject.

Preferred is a mammalian split-thickness skin graft material for use in the treatment of a subject with deficient and/or missing attached gingiva (keratinized gingiva) and/or mucosa according to the present invention, wherein the skin graft material is an allogeneic or xenogeneic material.

Preferred is a mammalian split-thickness skin graft material for use according to the present invention, wherein the skin graft material is porcine skin material. Usually, in this case the material is obtained either from wild-type and/or from genetically modified pigs. The material is prepared from the femur (e.g. joint or upper shell) and/or the hip, belly or back. The uppermost skin layer, the epidermis, is removed.

Suitable genetically modified and preferred materials for split-thickness skin grafts are disclosed in the literature, e.g. in Fischer and Schnieke (A. Xenotransplantation becoming reality. Transgenic Res 31, 391-398 (2022). https://doi.org/10.1007/s11248-022-00306-w). Of particular interest are materials comprising knockouts of genes like alpha Gal, neu5gc, SDA, and/or SLA, but also other modified materials can be preferably used.

The inventors surprisingly found that a non-autologous, e.g. porcine, split-thickness skin graft material has the ability to integrate into the surrounding tissue and to promote the growth of new, attached gingiva (keratinized gingiva) and/or mucosa through the body's healing processes.

The inventors also found that preferably porcine split-thickness skin is a cost-effective and readily available resource that imposes no additional burdens on the patient. The similar structure and composition of porcine split-thickness skin and human tissue reduces the risk of rejection reactions and complications. Therefore, this technology can be applied in periodontology and oral surgery for the treatment of gingival recession, periodontitis, and peri-implantitis as well as any other conditions that require regeneration of attached gingiva (keratinized gingiva) and/or mucosa.

The inventors were aware that attached gingiva is an epithelial tissue and then found that histologies of porcine skin also exhibited epithelial characteristics. Therefore, this material was used in the context of the present invention. Since autologous split-thickness skin works well for this indication (i.e. the provision of attached gingvia around dental implants/teeth), but the harvesting from a different part of the body requires an additional surgical procedure, having several risks and disadvantages (complications at the site of extraction, scar formation, need for second surgery, etc.) the inventors went to use porcine (xenogenic) split-thickness skin for this indication.

The term "material" as used in context of the present invention shall in particular relate to materials comprising biological cell material, such as living or dead cells, preferably living cells, as well as any secreted factors derived from the culturing of such cells. In certain preferred embodiments, the present invention relates to cell material comprising living biological cells.

In an even more preferred embodiment of the present invention, the term "material" is used to describe decellularized material such as decellularized donor split-thickness skin. Several techniques exist for decellularization with the majority based on detergent or enzymatic protocols which aim to eliminate all donor cells while preserving the mechanical properties of the remaining matrix. In the most common method, the donor material is first treated with various surfactants (soaps) and then rinsed several times so that all cells and chemical additives are removed and the basic structure of the connective tissue such as epithelial tissue for example attached gingiva (keratinized gingiva) and/or mucosa is preserved.

The process of decellularization should be performed while keeping the tissue as intact as possible. Decellularized tissues can also be commercially obtained.

The expression "biological cell" in context of the invention shall refer to a mammalian cell, most preferably a porcine cell. Certain embodiments of the invention pertain to epithelial cells included in the epidermis and cells from the layer of the dermis as preferred biological cells in context of the invention.

Hence, in certain preferred embodiments of the invention the cell material is an allogeneic or xenogeneic material, which is used to treat a subject. As used herein, the term "allogenic" or "allogeneic" refers to cells, tissues or organs that originate from other individuals of the same species. As used herein, the term "xenogenic" or "xenogeneic" refers to cells, tissues or organs that originate from individuals of different species. Such cells, tissues or organs are called xenografts and xenogeneic transplants.

In certain preferred embodiments the invention further includes the use of a splint (e.g. implant retained) that serves as scaffold material that is suitable for tissue regeneration. The term "scaffold material" is intended to refer to a compound that is capable of serving as a scaffold, for example for cells and cellular material as desired.

Preferred is a mammalian split-thickness skin graft material for use in the treatment of a subject with deficient and/or missing attached gingiva (keratinized gingiva) and/or mucosa according to the present invention, wherein the split-thickness skin graft material comprises the epithelial tissue from the epidermis and a portion of the dermis.

Further preferred is a mammalian split-thickness skin graft material for use in the treatment of a subject with deficient and/or missing attached gingiva (keratinized gingiva) and/or mucosa according to the present invention, wherein the treatment comprises a surgical procedure such as an insertion or attachment of the mammalian split-thickness skin graft material as reconstruction of the attached gingiva (keratinized gingiva) and/or mucosa of the subject.

Preferred is a mammalian split-thickness skin graft material for use in the treatment of a subject with deficient and/or missing attached gingiva (keratinized gingiva) and/or mucosa according to the present invention, wherein the surgical procedure is a vestibuloplasty with a subepthelial split flap preparation or any surgical procedure removing the movable mucosa or making space for the graft; for example removing epithelium or mucosa.

In the context of the present invention the term "vestibuloplasty" shall mean a surgery which allows the deepening of the vestibule and the increase of the keratinized/attached gingiva. It may turn out to be essential when a rehabilitation of an edentulous patient with a removable denture is going to be done on an alveolar ridge with a poor height, compromising the optimal retention and stability of the denture (15). The term "vestibuloplasty surgery" also includes subepithelial split flap preparation, or any surgical procedure removing the movable mucosa or making space for a graft by deepening the space between the cheeks and the teeth and/or by removing epithelium or mucosa.

Preferred is a mammalian split-thickness skin graft material for use in the treatment of a subject with deficient and/or missing attached gingiva (keratinized gingiva) according to the present invention, wherein the treatment is for a deficient or missing attached gingiva and/or mucosa having a thickness and/or depth (width) of less than 3 mm, preferably of less than 2 mm, and more preferably of less than 1 mm or for a missing attached gingiva (keratinized gingiva) and/or mucosa.

Preferred is a mammalian split-thickness skin graft material for use in the treatment of a subject with deficient and/or missing attached gingiva (keratinized gingiva) and/or mucosa according to the present invention, wherein the treatment is for promoting attached gingiva (keratinized gingiva) and/or mucosa growth or healing, such as for example to treat an attached gingiva (keratinized gingiva) and/or mucosa injury or disorder.

Preferred is a mammalian split-thickness skin graft material for use in the treatment of a subject with deficient and/or missing attached gingiva (keratinized gingiva) and/or mucosa according to the present invention, wherein the injury or disorder is selected from a gingiva/mucosa recession, periodontitis gingiva/mucosa trauma, an attached gingiva (keratinized gingiva)/mucosa deficit condition associated with tumour resection or oral mucosal disease, peri-implantitis, post-traumatic attached gingiva/mucosa surgery, post-prosthetic attached gingiva/mucosa surgery, post-plastic attached gingiva/mucosa surgery, post-dental implant surgery, chemotherapy treatment, Radiation therapy-induced gingiva/mucosa damage, post traumatic attached gingiva/mucosa loss, post-surgical attached gingiva/mucosa loss, post infectious attached gingiva/mucosa loss, attached gingiva/mucosa loss post incorrect allogeneic, autologous, synthetic or xenologous material incorporation, attached gingiva/mucosa loss because of burns, and attached gingiva/mucosa loss after alveolar bone loss and its reconstruction with allogeneic, autologous, synthetic or xenologous materials.

Preferred is a mammalian split-thickness skin graft material for use in the treatment of a subject with deficient and/or missing attached gingiva (keratinized gingiva) according to the present invention, wherein the subject is selected from a dog, cat, sheep, cow, goat, pig and human, preferably a human suffering from gingiva and/or mucosa injury or disorder.

In certain particular embodiments the mammalian split-thickness skin graft material of the invention is suitably preserved, such as cryopreserved, and this optionally requires thawing immediately before surgical insertion or attachment of the mammalian split-thickness skin graft material as reconstruction of the attached gingiva (keratinized gingiva) of the subject. The term "cryopreservation", as used herein, refers to the process of cooling and storing biological cells, tissues, or organs at low temperatures to maintain their viability. As a non-limiting example, cryopreservation can be the technology of cooling and storing cells at a temperature below the freezing point (e.g., -20°C or colder, -80°C or colder, or the like) that permits high rates of survivability of the cells upon thawing. In context of the invention the mammalian split-thickness skin graft material is ideally cryopreserved in order to provide an off-the-shelf product for implantation.

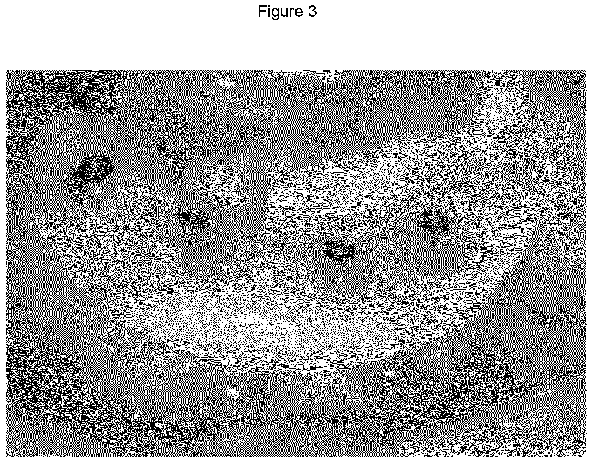

In some embodiments, the mammalian split-thickness skin graft has the shape of a cylinder, rectangle, trapezoid, is flat, convex or concave, and may have a channel formed on one or both sides thereof for an easy placement against a subject's tooth or implant, or for buttressing against a tooth or implant. In some embodiments, the mammalian split-thickness skin graft may be trimmed to size and snapped onto a rod by press fit with a prefabricated surgical tooth or implant-retained splint and/or by means of sutures (resorbable or non-resorbable) or tissue adhesive (see also Figure 3). In preferred embodiments, a prefabricated template is used that is fixed/slid onto the implant/tooth and covers the area and at the same time exerts a certain pressure onto the split-thickness skin transplant.

Preferred is a mammalian split-thickness skin graft material for use according to the present invention, wherein the split-thickness skin graft material has a width of between about 1 and 5 mm, preferably of between about 1 and 3 mm, and more preferably of about 3 mm. A particularly preferred thickness (tissue thickness) of the transplant (split-thickness skin) is at about 0.4-0.8 mm. For a tooth or implant, usually 1- 3 mm of attached width of gingiva are required in order to ensure a long-term survival.

According to a second aspect of the present invention the above object is solved by providing a therapeutic kit, comprising a container comprising the non-autologous mammalian split-thickness skin graft material for use according to the invention, together with additional components to preserve the skin graft.

Preferably, the therapeutic kit according to the invention additionally comprises useful components necessary for realizing a preservation of the graft. Buffers, media, or instructions for use are non-limiting examples of such additional components.

Several techniques for the preservation of skin grafts are known to a person skilled in the art and are published in Kearney, 2005 (16), but not limited thereto.

Since freezing a biological sample containing living material such as cells bears the risk of sheering and destruction of the cell material, cryopreservation medium is used in order to minimize the loss of cell material during the process. As used herein, the term "cryopreservation medium" or "cryoprotectant" is a substance that is used to protect eukaryotic cells (and also tissues, organs) from freezing damage. Further, the cryopreservation agent or cryoprotectant may protect the eukaryotic cells (and also tissues or organs) from cold and heat shock, dehydration, and cryo-toxicity during cryopreservation. The cryopreservation agent or cryoprotectant may be cell penetrating or non-penetrating. Non-limiting examples of cryoprotectants include glycerol, DMSO (dimethyl sulfoxide), propylene glycol, ethylene glycol, acetamide, and methanol. However, it should be cautioned that cryoprotectants have some disadvantages in that they can induce protein denaturation at higher temperature and cause cryoprotectant toxicity in cellular systems (like tissues and organs). The toxicity of the cryoprotectants is a major limitation to successful cryopreservation of eukaryotic cells. DSMO is a preferred cryoprotectant for use in accordance with the present invention.

Preferably, in some embodiments of the invention the cryopreservation is performed at 15°C or less, preferably is -20°C or less, preferably less than -50°C (about -70 °C or -80 °C); preferably the cryopreservation comprises a step of freezing at a freezing temperature of less than -20 °C, such as -40 °C or less, and subsequent storing of at temperatures higher than the freezing temperature.

According to a third aspect of the present invention the above object is solved by providing a method for the treatment of a disease, wherein the treatment comprises the administration of a mammalian split-thickness skin graft material to a subject in need of the treatment by adhering the mammalian split-thickness skin graft material to deficient attached gingiva/mucosa or by replacing missing attached gingiva/mucosa with mammalian split-thickness skin graft material.

According to a fourth aspect of the present invention, the above object is solved by providing a method for the treatment of a disease, wherein the treatment comprises surgical insertion or attachment of the mammalian split-thickness skin graft material according to the present invention for the reconstruction of the attached gingiva/mucosa of the subject.

Preferred is the method according to the present invention, wherein the treatment of a disease comprises promoting attached gingiva (keratinized gingiva) growth or healing, such as for example to treat a attached gingiva (keratinized gingiva) injury or disorder selected from a gingiva/mucosa recession, periodontitis gingiva/mucosa trauma, an attached gingiva (keratinized gingiva)/mucosa deficit condition associated with tumour resection, peri-implantitis, post-traumatic attached gingiva (keratinized gingiva)/mucosa surgery, post-prosthetic attached gingiva (keratinized gingiva)/mucosa surgery, post-plastic attached gingiva (keratinized gingiva)/mucosa surgery, post-dental implant surgery, chemotherapy treatment, post traumatic attached gingiva (keratinized gingiva)/mucosa loss, post-surgical attached gingiva (keratinized gingiva)/mucosa loss, post infectious attached gingiva (keratinized gingiva)/mucosa loss, attached gingiva (keratinized gingiva)/mucosa loss post incorrect allogeneic, autologous, synthetic or xenologous material incorporation, attached gingiva/mucosa loss because of burns, and attached gingiva (keratinized gingiva)/mucosa loss after alveolar bone loss and its reconstruction with allogeneic, autologous, synthetic or xenologous materials.

The terms "of the present invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

All references, patents, and publications cited herein are hereby incorporated by reference in their entirety.

In view of the above, it will be appreciated that the present invention also relates to the following itemized embodiments:
Item 1 A non-autologous mammalian split-thickness skin graft material for use in the treatment of a deficient and/or missing attached gingiva (keratinized gingiva) and/or mucosa in a subject.
Item 2 The mammalian split-thickness skin graft material for use according to item 1, wherein the skin graft material is an allogeneic or xenogeneic material.
Item 3 The mammalian split-thickness skin graft material for use according to item 1 or 2, wherein the skin graft material is porcine skin material.
Item 4 The mammalian split-thickness skin graft material for use according to any one of items 1 to 3, wherein the split-thickness skin graft material comprises the epithelial tissue from the epidermis and a portion of the dermis.
Item 5 The mammalian split-thickness skin graft material for use according to any one of items 1 to 4, wherein the treatment comprises a surgical procedure, such as an insertion, transplantation or attachment of the mammalian split-thickness skin graft material during reconstruction of the attached gingiva (keratinized gingiva) and/or mucosa of the subject.
Item 6 The mammalian split-thickness skin graft material for use according to item 5, wherein the surgical procedure is a vestibuloplasty.
Item 7 The mammalian split-thickness skin graft material for use according to any one of items 1 to 6, wherein the treatment is for a deficient attached gingiva (keratinized gingiva) and/or mucosa having a depth of less than 3 mm, preferably of less than 2 mm, and more preferably of less than 1 mm or for a missing attached gingiva (keratinized gingiva) and/or mucosa.
Item 8 The mammalian split-thickness skin graft material for use according to any one of items 1 to 7, wherein the treatment is for promoting the growth or healing of an attached gingiva (keratinized gingiva) and/or mucosa, such as, for example, for treating an attached gingiva (keratinized gingiva) and/or mucosa injury or disorder.
Item 9 The mammalian split-thickness skin graft material for use according to item 8, wherein the injury or disorder is selected from a gingiva/mucosa recession, periodontitis gingiva/mucosa trauma, an attached gingiva (keratinized gingiva)/mucosa deficit condition associated with tumour resection, peri-implantitis, post-traumatic attached gingiva (keratinized gingiva)/mucosa surgery, post-prosthetic attached gingiva (keratinized gingiva)/mucosa surgery, post-plastic attached gingiva (keratinized gingiva)/mucosa surgery, post-dental implant surgery, chemotherapy treatment, post traumatic attached gingiva (keratinized gingiva)/mucosa loss, post-surgical attached gingiva (keratinized gingiva)/mucosa loss, post infectious attached gingiva (keratinized gingiva)/mucosa loss, attached gingiva (keratinized gingiva)/mucosa loss post incorrect allogeneic, autologous or xenologous material incorporation, attached gingiva/mucosa loss because of burns, and attached gingiva (keratinized gingiva)/mucosa loss after alveolar bone loss and its reconstruction with allogeneic, autologous or xenologous materials.
Item 10 The mammalian split-thickness skin graft material for use according to any one of items 1 to 9, wherein the subject is selected from a dog, cat, sheep, cow, goat, pig, and human, preferably a human suffering from gingiva and/or mucosa injury or disorder.
Item 11 The mammalian split-thickness skin graft material for use according to any one of items 1 to 10, wherein the split-thickness skin graft material has a depth of at least 2 mm, more preferably of at least 2.5 mm, and even more preferably of at least 3 mm.
Item 12 A therapeutic kit, comprising a container comprising the non-autologous mammalian split-thickness skin graft material for use according to any one of items 1 to 11, together with additional components to preserve the skin graft.
Item 13 Use of the kit according to item 13 in the treatment of a deficient and/or missing attached gingiva (keratinized gingiva) and/or mucosa in a subject according to any one of items 1 to 11.
Item 14 A method for treating a deficient and/or missing attached gingiva and/or mucosa in a subject, comprising insertion, transplantation or attachment of a mammalian split-thickness skin graft material.
Item 15 The method according to Item 14, wherein the treatment is for promoting the growth or healing of an attached gingiva and/or mucosa, such as, for example, for treating an attached gingiva and/or mucosa injury or disorder, wherein preferably the injury or disorder is selected from a gingiva/mucosa recession, periodontitis gingiva/mucosa trauma, an attached gingiva/mucosa deficit condition associated with tumour resection, peri-implantitis, post-traumatic attached gingiva/mucosa surgery, post-prosthetic attached gingiva/mucosa surgery, post-plastic attached gingiva/mucosa surgery, post-dental implant surgery, chemotherapy treatment, post traumatic attached gingiva/mucosa loss, post-surgical attached gingiva/mucosa loss, post infectious attached gingiva/mucosa loss, attached gingiva/mucosa loss post incorrect allogeneic, autologous or xenologous material incorporation, attached gingiva/mucosa loss because of burns, and attached gingiva/mucosa loss after alveolar bone loss and its reconstruction with allogeneic, autologous or xenologous materialsduring reconstruction of the attached gingiva and/or mucosa of the subject.

**Figure 1****:** shows representative H&E staining pictures of native porcine split-thickness skin in 5x and 40x magnification.

**Figure 2****:** shows H&E staining pictures of healed attached gingiva and/or mucosa after (A) six weeks (6 W) and nine weeks (9 W) of the transplantation of a porcine split-thickness skin graft. (B) After eight weeks healing. The H&E staining pictures are shown in 5x and 40x magnification.

**Figure 3****:** shows a prefabricated implant-retained splint (according to Heberer and Nelson, 2009)

**Figure 4****:** shows an implant of autologous material together with porcine material according to the present invention (Figure 4C, shaded area). A) Implant after 12 weeks, B) implant after 7 months. No difference in the healing process can be seen between porcine and autologous material.

Certain aspects and embodiments of the invention will now be illustrated by way of example and with reference to the description and figures set out herein. Such examples of the methods, uses and other aspects of the present invention are representative only, and should not be taken to limit the scope of the present invention to only such representative examples.

### EXAMPLES

### Materials and Methods

**H&E staining.** Porcine split-thickness skin samples were fixed overnight in 10% formalin, washed with PBS and 70% ethanol, and embedded in paraffin. The paraffin blocks were cut into 4-6 µm sections using a microtome. Sections were then deparaffinized, rehydrated and stained with hematoxylin and eosin.

**Surgical procedure.** A vestibuloplasty was performed preparing a mucosal flap and reflecting to the lingual and buccal sites while sensitive structures such as the mental nerve were avoided. Possibly scar tissue was removed and the periosteum was left on the bone. Afterwards, the mammalian, preferably porcine, split-thickness skin graft was secured with sutures and a tooth or implant retained splint was fixed. The splint serves as a scaffold for tissue regeneration.

**Use of porcine split-thickness skin for promoting attached gingiva and/or mucosa growth.**

### Vestibuloplasty with Transplantation

### Step 1: Preparation of the Recipient Site

A split-thickness flap was prepared for the region where the attached gingiva was missing, and the flap was reflected to the lingual and buccal sites, while sensitive structures, such as the mental nerve, were avoided. The flap was divided into a superficial layer (mucosal flap) and the periosteum, while the periosteum needed to remain on the alveolar bone, as the integrity of this layer is essential for successful graft integration and healing. The mucosal flap was fixed with sutures at the periosteum to avoid flap mobility.

### Step 2: Transplantation

In this part, there was no need for harvesting an autologous graft from the patient. Instead, the aforementioned material according to the invention was used. This material was removed from its sterile packaging and promptly transferred to the recipient site in order to prevent contamination or dehydration. At the recipient site, the material was secured to the periosteum with sutures. It was pressed firmly enough to adhere to the periosteal layer, promoting proper integration. To ensure optimal fixation, custom-made protective devices crafted from medical-grade plastics may be used. These devices were secured to adjacent implants or remaining teeth, providing pressure on the graft and shielding it from mechanical forces during the healing phase.

### Step 3: Healing Process

Through the body's healing processes, the porcine split-thickness skin was integrated into the surrounding tissue and promoted the growth of new, attached gingiva (keratinized gingiva). Sutures were removed after 10-14 days.

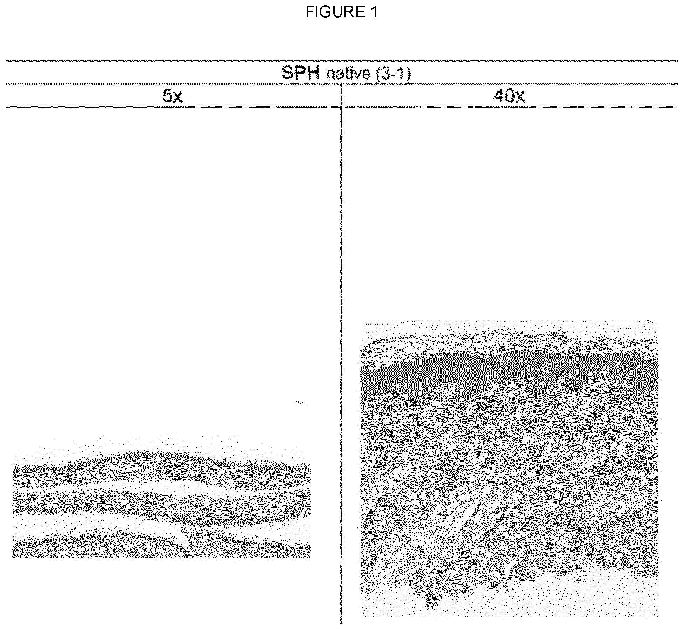

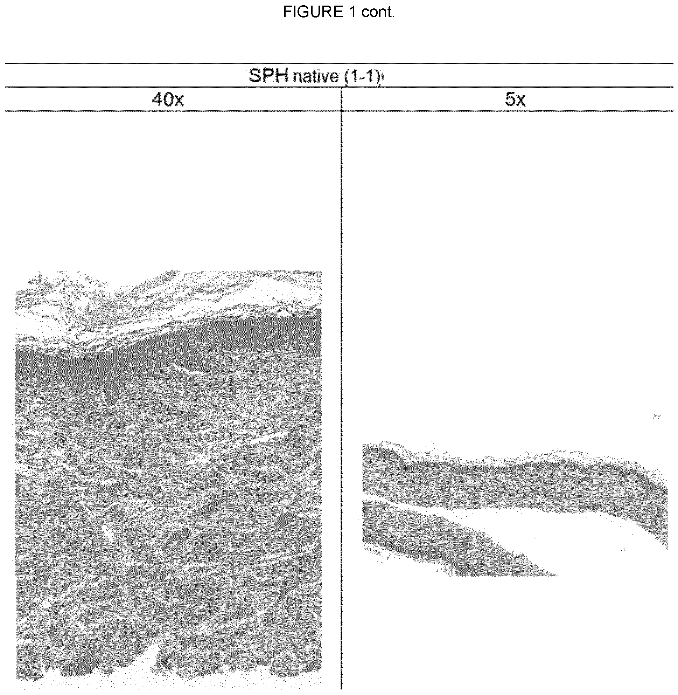

Figure 1 shows the native structure of porcine split-thickness skin, and the structure reveals a suitability of the porcine split-thickness skin as a mammalian split-thickness skin graft material for use in the treatment of a subject with deficient and/or missing attached gingiva and/or mucosa.

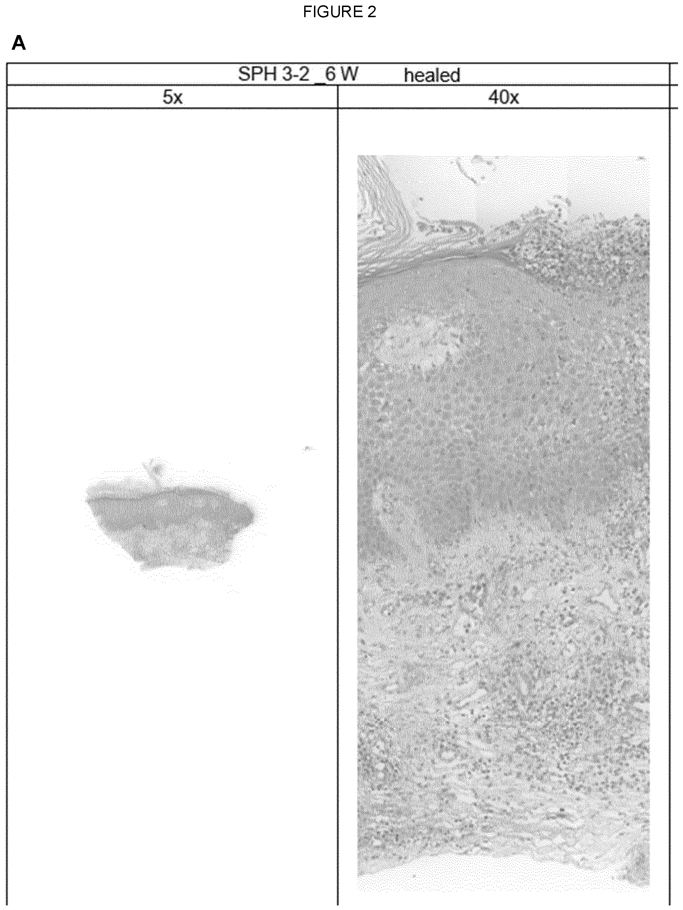

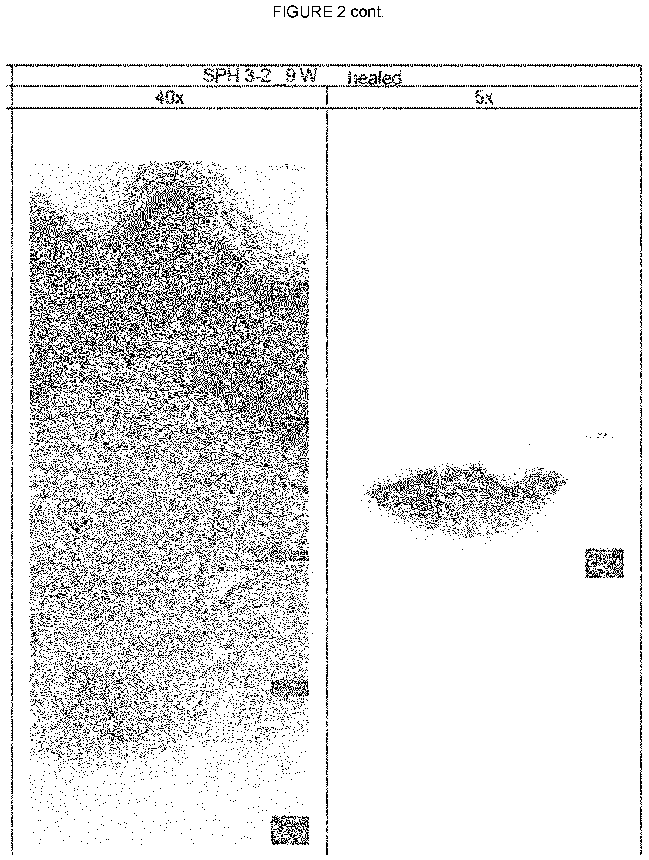

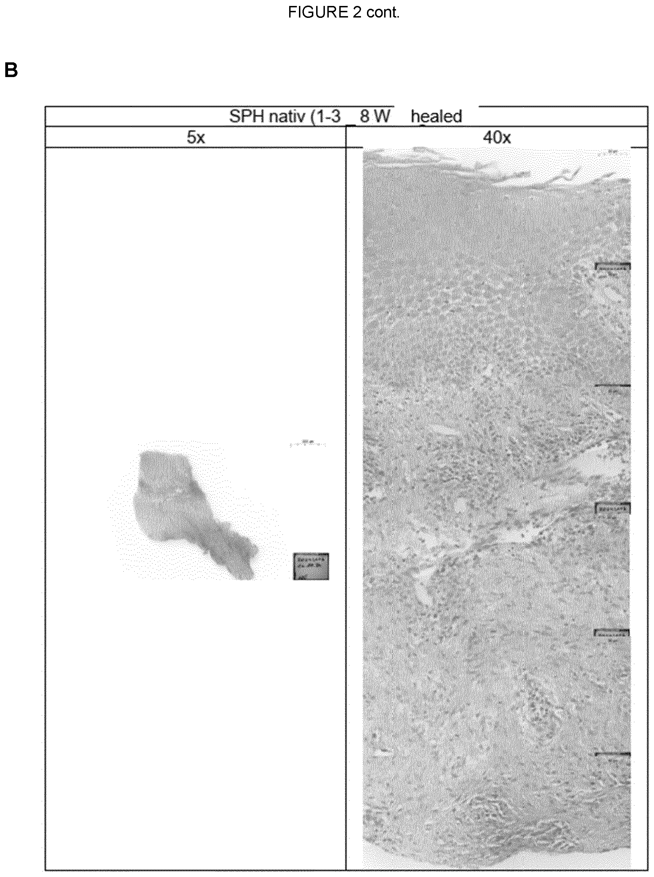

Figure 2 shows that after a healing period of six to nine weeks after surgery (here vestibuloplasty), the porcine split-thickness skin graft fully promotes or replaces a deficient or missing attached gingiva and/or mucosa.

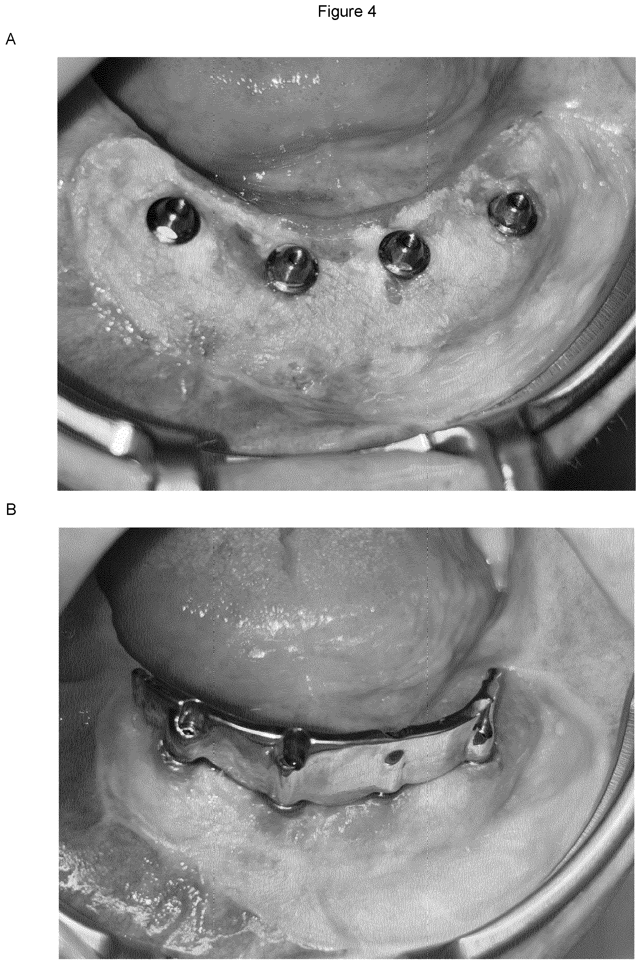

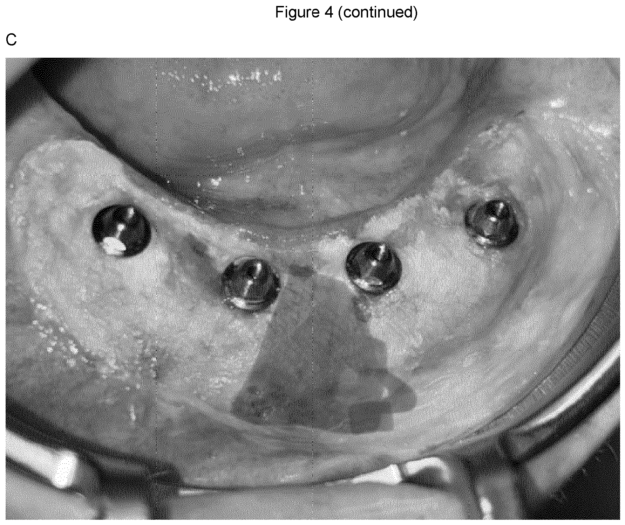

Figure 4 also shows that after a healing period of 12 weeks and 7 months after surgery, the porcine split-thickness skin graft fully promotes or replaces a deficient or missing attached gingiva and/or mucosa.
It was therefore surprisingly found that the process resulted in an at least good healing, as was characterized by, in particular, a combination of optimal vascularization, fast epithelial formation, lack of inflammation and infection, acceptable scar formation/shrinking, and sufficient aesthetic results, when compared to other methods.

### REFERENCES

1. Tarnow D, Hochman M, Chu S, Fletcher P. A New Definition of Attached Gingiva Around Teeth and Implants in Healthy and Diseased Sites. Int J Periodontics Restorative Dent. 2021 Jan-Feb; 41(1):43-49.
2. Ladwein C, et al. Is the presence of keratinized mucosa associated with periimplant tissue health? A clinical cross-sectional analysis. Int J Implant Dent. 2015 Dec, 1:11.
3. Mahardawi B, et al. The lack of keratinized mucosa as a risk factor for peri-implantitis: a systematic review and meta-analysis. Sci Rep. 2023 Mar 7;13(1):3778.
4. Ramanauskaite A, Schwarz F, Sader R. Influence of width of keratinized tissue on the prevalence of peri-implant diseases: A systematic review and meta-analysis. Clin Oral Implants Res. 2022 Jun; 33 Suppl 23:8-31.
5. Thoma DS, Buranawat B, Hammerle CH, Held U, Jung RE. Efficacy of soft tissue augmentation around dental implants and in partially edentulous areas: a systematic review. J Clin Periodontol. 2014; 41 Suppl 15:S77-91.
6. Heberer S, et al. Histomorphometric analysis of extraction sockets augmented with Bio-Oss Collagen after a 6-week healing period: A prospective study. Clin Oral Implants Res. 2008 Dec; 19(12):1219-25.
7. Bassetti RG, Stähli A, Bassetti MA, Sculean A. Soft tissue augmentation procedures at second-stage surgery: a systematic review. Clin Oral Investig. 2016 Sep; 20(7):1369-87.
8. Bertl K, Melchard M, Pandis N, Muller-Kern, Stavropoulos A. Soft tissue substitutes in non-root coverage procedures: a systematic review and meta-analysis Clin Oral Investig. 2017; 21(2):505-518.
9. Slanetz CA, Rankow RM. The intra-oral use of split-thickness skin grafts in head and neck surgery. The American Journal of Surgery 1962; 104(5):721-726.
10. Fang W, et al. A new submerged split-thickness skin graft technique to rebuild peri-implant keratinized soft tissue in composite flap reconstructed mandible or maxilla. Oral Surgery, Oral Medicine, Oral Pathology and Oral Radiology 2012 Mar; 113(3):e4-e9.
11. Mosshammer E. Einsatz von Hautersatzmaterialien in der Plastischen Chirurgie mit Schwerpunkt auf den Einsatz von Xenografts. Thesis: Klinische Abteilung für plastische, ästhetische undrekonstruktive Chirurgie, Universität Graz Austria 2015.
12. Yamamoto T, Iwase H, King TW, Hara H, Cooper DKC. Skin xenotransplantation: Historical review and clinical potential Burns 2018 Vol. 44 Issue 7 Pages 1738-1749
13. Friedmann E. A preliminary report on pigskin grafts in oral surgery. Int J Oral Surg. 1974; 3:269-272.
14. Kaspar DW, Laskin DM. The effect of porcine skin and autogenous epithelial grafts on the contraction of experimental oral wounds. J Oral Maxillofac Surg. 1983 Mar; 41(3):143-52.
15. Fokam ST, et al. Vestibuloplasty to enhance denture stability: About two observations. Advances in Oral and Maxillofacial Surgery. 2022; 6: 100288.
16. Kearney JN. Clin Dermatol. 2005 Jul 15;23(4):357-364. doi: 10.1016/j.clindermatol.2004.07.018.

## Claims

1. A non-autologous mammalian split-thickness skin graft material for use in the treatment of a deficient and/or missing attached gingiva and/or mucosa in a subject.

2. The mammalian split-thickness skin graft material for use according to claim 1, wherein the skin graft material is an allogeneic or xenogeneic material.

3. The mammalian split-thickness skin graft material for use according to claim 1 or 2, wherein the skin graft material is porcine skin material.

4. The mammalian split-thickness skin graft material for use according to any one of claims 1 to 3, wherein the split-thickness skin graft material comprises the epithelial tissue from the epidermis and a portion of the dermis.

5. The mammalian split-thickness skin graft material for use according to any one of claims 1 to 4, wherein the treatment comprises a surgical procedure, such as an insertion, transplantation or attachment of the mammalian split-thickness skin graft material during reconstruction of the attached gingiva and/or mucosa of the subject.

6. The mammalian split-thickness skin graft material for use according to claim 5, wherein the surgical procedure is a vestibuloplasty.

7. The mammalian split-thickness skin graft material for use according to any one of claims 1 to 6, wherein the treatment is for a deficient attached gingiva and/or mucosa having a depth of less than 3 mm, preferably of less than 2 mm, and more preferably of less than 1 mm or for a missing attached gingiva and/or mucosa.

8. The mammalian split-thickness skin graft material for use according to any one of claims 1 to 7, wherein the treatment is for promoting the growth or healing of an attached gingiva and/or mucosa, such as, for example, for treating an attached gingiva and/or mucosa injury or disorder.

9. The mammalian split-thickness skin graft material for use according to claim 8, wherein the injury or disorder is selected from a gingiva/mucosa recession, periodontitis gingiva/mucosa trauma, an attached gingiva/mucosa deficit condition associated with tumour resection, peri-implantitis, post-traumatic attached gingiva/mucosa surgery, post-prosthetic attached gingiva/mucosa surgery, post-plastic attached gingiva/mucosa surgery, post-dental implant surgery, chemotherapy treatment, post traumatic attached gingiva/mucosa loss, post-surgical attached gingiva/mucosa loss, post infectious attached gingiva/mucosa loss, attached gingiva/mucosa loss post incorrect allogeneic, attached gingiva/mucosa loss because of burns, autologous or xenologous material incorporation, and attached gingiva/mucosa loss after alveolar bone loss, and its reconstruction with allogeneic, autologous or xenologous materials.

10. The mammalian split-thickness skin graft material for use according to any one of claims 1 to 9, wherein the subject is selected from a dog, cat, sheep, cow, goat, pig, and human, preferably a human suffering from gingiva and/or mucosa injury or disorder.

11. The mammalian split-thickness skin graft material for use according to any one of claims 1 to 10, wherein the split-thickness skin graft material has a depth of at least 2 mm, more preferably of at least 2.5 mm, and even more preferably of at least 3 mm.

12. A therapeutic kit, comprising a container comprising the non-autologous mammalian split-thickness skin graft material for use according to any one of claims 1 to 11, together with additional components to preserve the skin graft.

13. Use of the kit according to claim 13 in the treatment of a deficient and/or missing attached gingiva and/or mucosa in a subject according to any one of claims 1 to 11.

14. A method for treating a deficient and/or missing attached gingiva and/or mucosa in a subject, comprising insertion, transplantation or attachment of a mammalian split-thickness skin graft material.

15. The method according to claim 14, wherein the treatment is for promoting the growth or healing of an attached gingiva and/or mucosa, such as, for example, for treating an attached gingiva and/or mucosa injury or disorder, wherein preferably the injury or disorder is selected from a gingiva/mucosa recession, periodontitis gingiva/mucosa trauma, an attached gingiva/mucosa deficit condition associated with tumour resection, peri-implantitis, post-traumatic attached gingiva/mucosa surgery, post-prosthetic attached gingiva/mucosa surgery, post-plastic attached gingiva/mucosa surgery, post-dental implant surgery, chemotherapy treatment, post traumatic attached gingiva/mucosa loss, post-surgical attached gingiva/mucosa loss, post infectious attached gingiva/mucosa loss, attached gingiva/mucosa loss post incorrect allogeneic, autologous or xenologous material incorporation, attached gingiva/mucosa loss because of burns, and attached gingiva/mucosa loss after alveolar bone loss and its reconstruction with allogeneic, autologous or xenologous materialsduring reconstruction of the attached gingiva and/or mucosa of the subject.
